Europäisches Patentamt

European Patent Office

Office européen des brevets

(11) Veröffentlichungsnummer: **0 298 400**
**A1**

## (12) EUROPÄISCHE PATENTANMELDUNG

(21) Anmeldenummer: 88110613.2

(51) Int. Cl.4: **A61B 17/58**

(22) Anmeldetag: 02.07.88

(30) Priorität: 08.07.87 DE 3722538

(43) Veröffentlichungstag der Anmeldung:
11.01.89 Patentblatt 89/02

(84) Benannte Vertragsstaaten:
AT BE CH DE ES FR GB IT LI LU NL SE

(71) Anmelder: Wasserstein, Isidor, Prof. Dr. med.
Kurt-Schumacher-Allee 17
D-2800 Bremen 41(DE)

(72) Erfinder: Wasserstein, Isidor, Prof. Dr. med.
Kurt-Schumacher-Allee 17
D-2800 Bremen 41(DE)

(74) Vertreter: Funck-Hartherz, Anna-Eleonore,
Dipl.-Phys.
Hoherodskopfstrasse 41
D-6000 Frankfurt (Main) 50(DE)

(54) Vorrichtung zur Fixierung von Knochensegmenten.

(57) Die Erfindung bezieht sich auf eine Vorrichtung zur Reposition und Fixierung sowie Kompression von Knochensegmenten unter Verwendung eines Kirschnerdrahtes, also eines Drahtes für knochenchirurgische Eingriffe, der mit einer geeigneten Spitze ausgebildet ist. Die Befestigung solcher Drähte nach Reposition, Fixierung oder Kompression des Knochens sowie die Spannung solcher Drähte erfolgt über Spannhalter außerhalb der Gliedmaße.

Die Erfindung bezieht sich darauf, den Kirschnerdraht so auszustatten, daß er innerhalb der Weichteile bzw. des Muskelgewebes angeordnet und auch gespannt werden kann, so daß keine äußeren Spannvorrichtungen erforderlich sind.

Die erfindungsgemäße Vorrichtung besteht aus einem Kirschnerdraht, auf dem eine oder mehrere Drehgelenkstützen befestigbar aufgezogen sind.

Fig. 6

EP 0 298 400 A1

## Vorrichtung zur Fixierung von Knochensegmenten

Die Erfindung bezieht sich auf eine Vorrichtung zur Reposition und Fixierung sowie Kompression von Knochensegmenten unter Verwendung eines Kirschnerdrahtes. Unter Kirschnerdrähten werden Drähte für knochenchirurgische Eingriffe verstanden, die mit einer geeigneten Spitze ausgebildet sind. Die Befestigung der Drähte nach Reposition des Knochens sowie deren Spannung erfolgt über Spannhalter außerhalb der Gliedmaße.

Die der Erfindung zugrundeliegende Aufgabe besteht in der Schaffung einer Vorrichtung unter Verwendung eines Kirschnerdrahtes zur Reposition und Fixation einer Insultstelle. Die erfindungsgemäße Vorrichtung soll in der Nähe oder an der Insultstelle ansetzbar sein. Die Aufgabe der Erfindung erstreckt sich auch darauf, die Vorrichtung innerhalb der Weichteile bzw. des Muskelgewebes anzuordnen und zu spannen. Bei Erforderung großer Stabilität kann die Vorrichtung auch von außen vorzugsweise mittels eines Ringfixateurs einer Spannung unterworfen werden.

Zur Lösung dieser Aufgabe wird eine Vorrichtung unter Verwendung eines Kirschnerdrahtes vorgesehen, die dadurch gekennzeichnet ist, daß auf dem Kirschnerdraht eine oder mehrere Drehgelenkstützen befestigbar aufgezogen sind. Diese Drehgelenkstützen bestehen aus einer Platte mit einer Kugel, wobei erstere einen kreuzförmigen Schlitz aufweist und ein- oder beidseitig eine kalottenförmige Vertiefung besitzt. Die Kugel ist dabei in der Regel kleiner als die Platte und zur Durchführung des Drahtes durchbohrt.

Zufolge des erfindungsgemäß ausgebildeten Drehgelenkes mit Platte und Kugel in Verbindung mit dem Kreuzschlitz wird ermöglicht, daß die Platte einen weiten Spielraum an Stellungen fast bis zur Parallel-Lage gegenüber dem Kirschnerdraht einnehmen kann, so daß stets eine weiche, den Patienten wenig belastende Anlage der Platte des Gelenks an der Knochenhaut oder Epidermis nächst der Austrittsstelle des Kirschnerdrahtes erreicht wird und zwar unabhängig davon, in welchem Winkel der Kirschnerdraht den verletzten Knochen und damit das diesen umgebende Gewebe durchsetzt. Die in die kalottenförmige Vertiefung der Platte eingreifende Kugel ermöglicht durch Schwenkung der Platte eine den Gegebenheiten entsprechende Anlage an die Knochenhautpartie wie auch gegebenenfalls an die Epidermis und vermeidet eine Verschiebung der Platte nach Abstützung und Fixierung der Drehgelenkstütze. Die bewegliche Platte mit abgerundetem Rand vermeidet Druckstellen oder Beschädigungen der Knochenhaut und den damit Hand in Hand gehenden Nachteil der gestörten Blutversorgung des Knochens. Die Fixierung der erfindungsgemäßen Vorrichtung nach Reposition und Spannung kann durch einfaches Abquetschen des Drahtes, rechtwinkliges Abbiegen des Drahtes oder durch das Aufziehen bekannter Stecksicherungen erfolgen.

Es besteht die Möglichkeit, die aus Kugel und Platten bestehende Drehgelenkstütze entsprechend den Erfordernissen an einer Seite oder an beiden Seiten der Insultstelle anzuordnen. Im letzteren Fall erfolgt eine starre Fixation. Häufig ist auch die Verwendung von einem Drehgelenk an der einen Seite und einer Gegenfeder an der anderen Seite zweckmäßig, gleichgültig ob diese auf den Draht aufgesetzt oder mit dem Draht gewickelt wird. Auf diese Weise werden die Mikrobewegungen zufolge der Elastizität der Feder zugelassen, die sich vorteilhaft auf das Heilungsgeschehen auswirken. Deshalb besteht eine bevorzugte Ausführung darin, daß auf der einen Seite der Insultstelle die geschilderte Drehgelenkstütze und auf der anderen Seite der Insultstelle eine Feder fixiert wird.

Bei Verletzungen kleinerer Gliedmaßen, z.B. Finger, reicht auch das Aufsetzen einer an der Epidermis anliegenden Sperrkugel (anstelle der vorerwähnten Feder) auf den Kirschnerdraht aus, die entweder aus Kunststoff oder Metall besteht. Im ersteren Fall ermöglicht die Eigenelastizität der Kugel einen festen Sitz auf dem Draht, während sie im zweiten Fall mit einem der vorerwähnten Mittel auf dem Draht gehalten und gegebenenfalls gespannt wird. In vorgenannten Fällen sorgt die Eigenelastizität des Gewebes für die zur Heilung notwendigen Mikrobewegungen.

Es hat sich gezeigt, daß es mit der erfindungsgemäßen Vorrichtung erstmals möglich ist, mit einem Kirschnerdraht im Gebiet der Knochenweichteile bzw. des Muskelgebietes eine Spannung, auch eine Dauerspannung, ohne äußere Fixateure zu erzielen. Diese Tatsache ist nicht nur bei der Behandlung von Knochenfrakturen, sondern auch bei der Behandlung von Bänderverletzungen und -rissen von großer Wichtigkeit, die bisher nur durch Eingipsen, Einsetzen von Schrauben oder ungespannten Kirschnerdrähten behandelt werden konnten.

Eine Indikation für die Behandlung mit der erfindungsgemäßen Vorrichtung ergibt sich bei allen Knochen- und Bänderverletzungen besonders bei Kindern und alten Patienten, da die Vorrichtung den Vorteil bietet, daß deren Anwendung ohne große Narkose, ohne großen Blutverlust und ohne, daß die Bewegungsfreiheit benachbarter Gelenke eingeschränkt wird, erfolgen kann. Außerdem sind die Patienten auch bei Beinverletzungen schneller wieder gehfähig. Wird eine zusätzliche Stabilität erforderlich, so wird der Kirschnerdraht mit einem Fixateur, z.B. einem Ringfixateur, gespannt, der eine Vermeidung jeglicher Verschiebungen gewährleistet.

Weitere Einzelheiten der Erfindung sind in der beigefügten Zeichnung dargestellt. Dabei zeigt

Fig. 1 eine Ansicht der Vorrichtung in Explosivdarstellung,

Fig. 2 eine vervollständigte Ansicht entsprechend Fig. 1,

Fig. 3a, 3b Vorrichtungen gemäß der Erfindung in zusammengebautem Zustand,

Fig. 4, 5 die erfindungsgemäße Vorrichtung an einer Insultstelle,

Fig. 6 - 9 weitere Beispiele für die Anwendung der Vorrichtung gemäß der Erfindung.

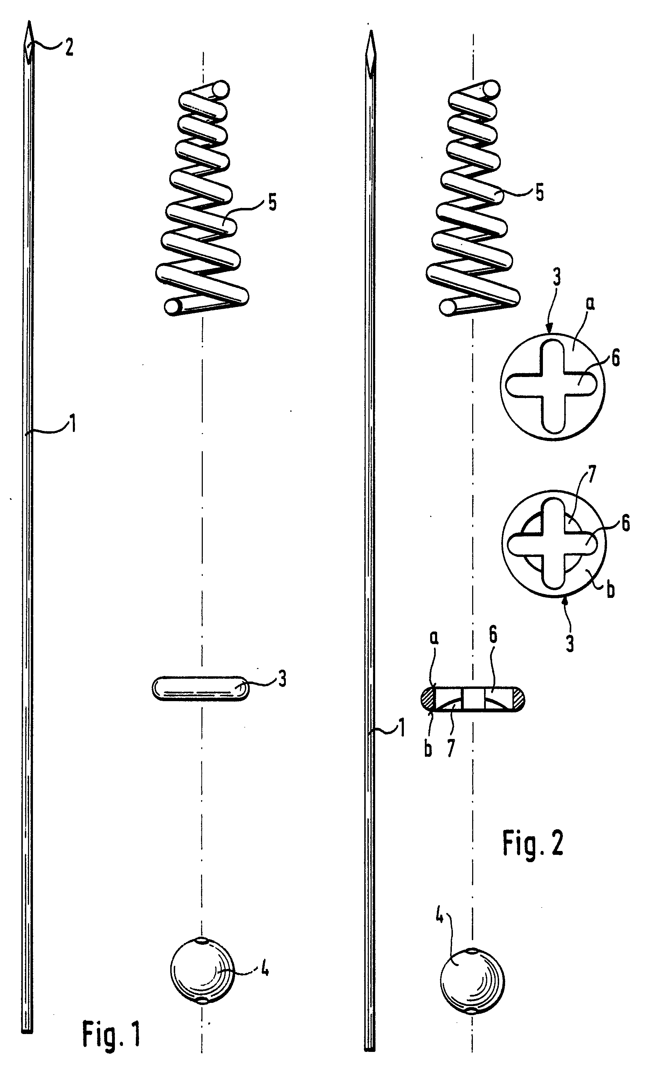

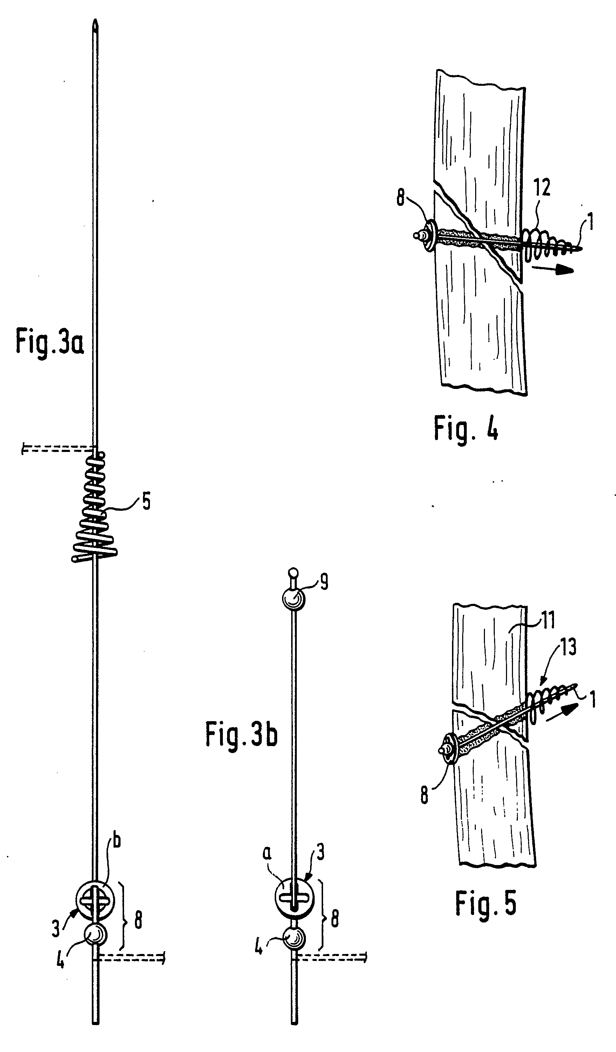

In Fig. 1 ist ein Kirschnerdraht 1 mit Lanzenspitze 2 gezeigt und die aus der Platte 3 und der Kugel 4 bestehende Drehgelenkstütze 8 (siehe Fig. 3a und 3b) sowie eine Stützfeder 5, die anstelle einer Drehgelenkstütze an einer der Austrittsstellen des Kirschnerdrahtes zur Abstützung vorgesehen ist. Der Rand der Platte ist abgerundet.

Ebenfalls eine Explosivdarstellung zeigt Fig. 2, die jedoch um Draufsichten auf die Plattenseiten ergänzt ist. Die Platte 3 besitzt einen kreuzförmigen Schlitz 6, der dafür sorgt, daß die Platte 3 gegenüber dem durch den Schlitz geführten Kirschnerdraht 1 fast bis in eine Parallel-Lage schwenkbar ist, was der erfindungsgemäßen Vorrichtung ermöglicht, eine Anpassung an jede Knochenhautpartie und an jede Schrägführung des Drahtes durch die Insultstelle zu erreichen. Damit wird eine druckfreie Anlage an die Knochenhaut und eine gute Durchblutung gesichert. Auch wird dadurch eine Verletzung der Knochenhaut ausgeschaltet und damit eine Osteolyse sowie Knochennekrose vermieden. Aus diesem Grund ist auch der Plattenrand abgerundet, also ohne scharfe Kanten gehalten. Die Oberseite a der Platte in Bezug auf die Zeichnung gesehen ist glatt und zeigt lediglich den kreuzförmigen Schlitz, während die Unterseite b eine Vertiefung in Art einer Kalotte für die durchbohrte Kugel 4 zeigt. Die Kugel 4 bildet mit der Platte eine Drehgelenkstütze, die den Austritt des Einschlagdrahtes fixiert und für eine druckfreie Abstützung des Drahtes an der Austrittsstelle der Haut sorgt.

In den Figuren 3a und 3b ist die erfindungsgemäße Vorrichtung vollständig nach Aufzug auf den Einschlagdraht 1 gezeigt. Auf den Einschlagdraht 1 ist die aus Platte und Kugel bestehende Drehgelenkstütze 8 aufgezogen. Auf der von den Drehgelenkstützen abgewandten Seite der Insultstelle befindet sich die Feder 5 oder eine Sperrkugel 9. Zur Fixierung der Abstützung und gegebenenfalls nach Spannung des Einschlagdrahtes werden die Enden der Drähte einfach unter Breitdrücken abgequetscht oder rechtwinkelig abgebogen, wie es gestrichelt in den Figuren dargestellt ist. Wie in Fig. 3a dargestellt, ist die Feder 5 auf den Draht aufgeschoben. Anstelle davon kann auch der Draht selbst als Kegelfeder gebogen sein. Zur Fixierung der erfindungsgemäßen Vorrichtung kann auch eine Stecksicherung aufgeschoben werden, die die Feder in der gewünschten Stellung hält. Die Feder ermöglicht, daß heilungsfördernde Mikrobewegungen an der Insultstelle zugelassen werden. Die Sperrkugel 9 (vgl. Fig. 3) kann aus Kunststoff oder Metall bestehen, wobei möglicherweise die Sperrkugel zufolge ihrer Eigenelastizität auf dem Draht gehalten ist oder durch eine in die Kugel einschraubbare Sicherungsschraube. Auch kann die Kugel, wie in der Figur gezeigt, durch Breitquetschen des Drahtes und Abzwicken gesichert werden. Die Verwendung der Sperrkugel wird meist bei Verletzungen kleinerer Gliedmaße, wie z.B. Finger, herangezogen, da dann die Elastizität der Epidermis und des Gewebes ausreicht, um die Mikrobewegungen zuzulassen.

In den Figuren 4 und 5 ist die erfindungsgemäße Vorrichtung am Knochen 11 gezeigt. Nach Reposition werden die Kirschnerdrähte 1 durch die Insultstelle gezogen und die Drehgelenkstütze aufgezogen, die die Abstützung auf der Knochenhaut respektive dem Gewebe bildet. Hinter dem Gelenk ist der Draht abgequetscht. Wie in Fig. 4 gezeigt, wird auf der der Drehgelenkstütze gegenüberliegenden Seite der Kirschnerdraht zu einer Kegelfeder 12 - es kann auch eine Schrauben- oder Spiralfeder sein - aufgewickelt oder aber, wie in Fig. 5 gezeigt, eine Kegelfeder 5 oder dergl. aufgeschoben und durch eine Stecksicherung 13 in Abstützung an der Knochenhaut gehalten. Selbstverständlich kann auch dabei anstelle der Stecksicherung der Kirschnerdraht einfach abgequetscht oder rechtwinkelig abgebogen werden. In den Figuren 4 und 5 sind die Kirschnerdrähte in verschiedenen Winkeln zur Längsachse des Knochens durch die Insultstelle durchgezogen. Unabhängig von dem Durchzugwinkel liegt das Abstützungsgelenk 8 flach im Bereich der Drahtaustrittsstelle an der Haut an und sorgt somit für eine einwandfreie und sichere Abstützung des Kirschnerdrahtes nach Reposition und gegebenenfalls Spannung.

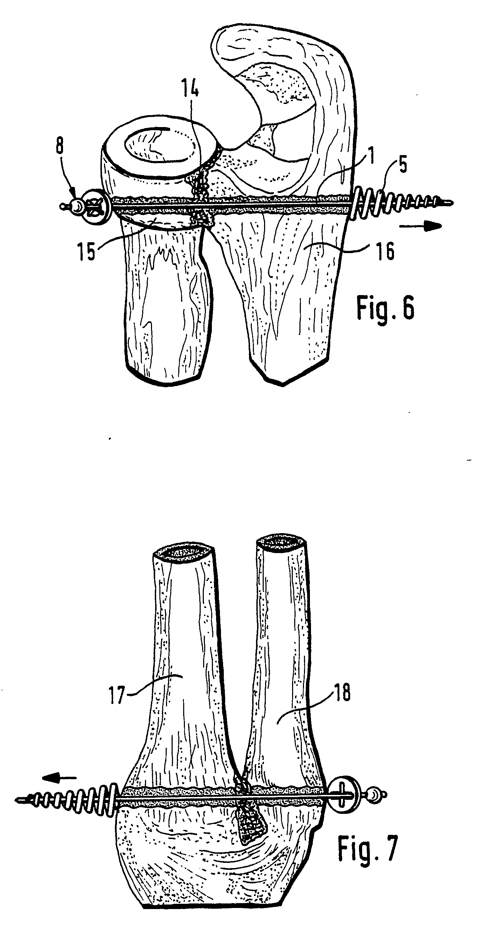

In den Figuren 6 bis 9 sind Anwendungsbeispiele der erfindungsgemäßen Vorrichtung dargestellt.

Fig. 6 zeigt die erfindungsgemäße Vorrichtung nach Reposition und Fixation eines radioulnaren Bandrisses am Ellenbogengelenk. Dabei ist das Band 14 zwischen Speichenköpfchen 15 und Ellenbogen 16 verletzt. Durch das Insultgebiet ist der Kirschnerdraht 1 gezogen, und die Drehgelenkstütze 8 legt sich in der Gegend des Speichenköpfchens an der Knochenhaut unmittelbar an, während auf das andere Austrittsende des Drahtes 1 eine Kegelfeder 5 aufgezogen ist, die sich am Ellenbogenknochen abstützt. Die von außen in Richtung des Pfeiles erfolgte Spannung des Kirschnerdrahtes wird über die Feder 5 aufrechterhalten, die auf geeignete Weise mit dem Draht 1 verbunden ist. Durch die Kegelfeder werden Mikrobewegungen an der Insultstelle zugelassen.

Die Behandlung eines Bandrisses am Handgelenk zwischen Elle 17 und Speiche 18 mittels der erfindungsgemäßen Vorrichtung - in gleicher Weise wie in Fig. 6 - ist in Fig. 7 dargestellt. In den letztgenannten Fällen ist die Belastung nur gering, so daß die erfindungsgemäße Vorrichtung allein ohne zusätzlichen Fixateur die eine Verschiebung vermeidende Stabilisierung gewährleistet.

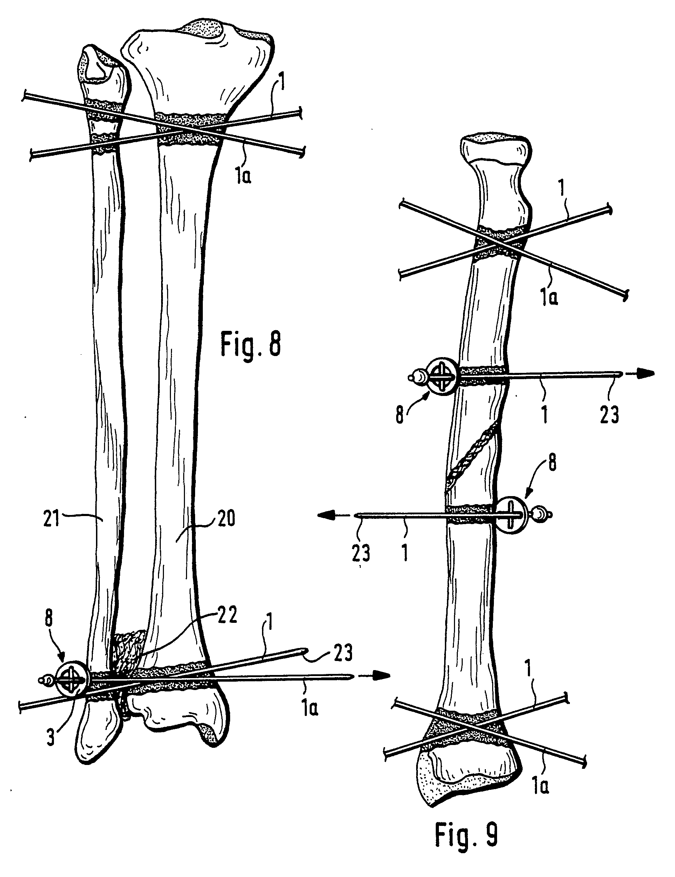

In den Figuren 8 und 9 sind Verletzungszustände gezeigt, die zur Stabilisierung außer der erfindungsgemäßen Vorrichtung einen zusätzlichen Ringfixateur benötigen.

In Fig. 8 ist ein Schien- 20 und Wadenbein 21 gezeigt nach Zustand eines fußgelenksnahen Bandrisses. Der Kirschnerdraht 1 der erfindungsgemäßen Vorrichtung ist durch die Insultstelle 22 gezogen, und die Platte 3 des Drehgelenks 8 legt sich unmittelbar an das Wadenbein an. Da die Belastung eines Beins stark ist, ist es unerläßlich, zur Stabilisierung zusätzlich einen Ringfixateur vorzusehen. Das freie Ende 23 des Kirschnerdrahtes 1 wird an einem der Ringe eines Ringfixateurs unter Spannen befestigt. Außerdem ist ein zweiter Draht 1a durch die Insultstelle geführt und am gleichen Ring befestigt. Um der Belastung genügend Rechnung tragen zu können, sind zwei weitere gekreuzte Drähte 1 und 1a kniegelenknah durch das Waden- 21 und Schienbein 20 gezogen.

In Fig. 9 ist die erfindungsgemäße Vorrichtung bei einer Speichenschrägfraktur schematisch dargestellt. Beidseitig der Frakturstelle sind Kirschnerdrähte 1 der erfindungsgemäßen Vorrichtung durchgezogen und über Drehgelenke abgestützt, jedoch erfolgt dabei die Abstützung auf gegenüberliegenden Seiten des Knochens, um bei Spannung eine Kompression der Knochensegmente an der Bruchstelle zu erhalten. Die freien Enden 23 der Drähte sind wiederum an einem schräggestellten Ring des Ringfixateurs oder an zwei Ringen befestigt. Wegen der bei Schrägbrüchen leicht eintretenden Verschiebung der zu fixierenden Teile sind nächst dem Hand- und Ellenbogengelenk noch gekreuzte Kirschnerdrähte 1a vorgesehen und an nicht dargestellten Ringen in bekannter Weise befestigt.

Die Vorrichtung gemäß der Erfindung ist überall dort einsetzbar, wo Bänderläsionen, Knochenfrakturen und Knochenzertrümmerungen vorkommen und ermöglicht bei einem schonenden Eingriff auf einfache und zweckmäßige Weise eine stabile Fixierung und Spannung des Kirschnerdrahtes auch im Bereich der Weichteile bzw. des Muskelgebietes nach Reposition der insultierten Gliedmaßen, und zwar gleichgültig, in welchem Neigungswinkel der Draht durch die Insultstelle geführt ist, und ohne daß Gefahr einer Knochenhautverletzung besteht. Sind die verletzten Gliedmaße unbelastet, so kann die erfindungsgemäße Vorrichtung ohne zusätzliche Hilfsmittel verwendet werden. Werden die verletzten Gliedmaße Belastungen ausgesetzt, so ist die zusätzliche Anwendung von Fixateuren, vorzugsweise Ringfixateuren, zur Stabilisierung erforderlich, um Verschiebungen zu vermeiden. Darüber hinaus ermöglicht die erfindungsgemäße Vorrichtung Mikrobewegungen an der Insultstelle, die für die Heilungstendenz so bedeutsam sind.

## Ansprüche

1.) Vorrichtung zur Reposition und Fixierung von Knochensegmenten unter Verwendung eines Kirschnerdrahtes,
**dadurch gekennzeichnet,**
daß auf dem Kirschnerdraht (1) eine oder mehrere Drehgelenkstützen (8) befestigbar aufgezogen sind.

2.) Vorrichtung nach Anspruch 1,
**dadurch gekennzeichnet,**
daß die Drehgelenkstütze (8) aus einer Platte (3) und einer Kugel (4) besteht.

3.) Vorrichtung nach Anspruch 2,
**dadurch gekennzeichnet,**
daß die Platte (3) einen kreuzförmigen Schlitz (6) aufweist.

4.) Vorrichtung nach Anspruch 2 oder 3,
**dadurch gekennzeichnet,**
daß die Platte (3) ein- oder beidseitig eine kalottenförmige Vertiefung (7) besitzt.

5.) Vorrichtung nach Anspruch 1,
**dadurch gekennzeichnet,**
daß anstelle von einem der Drehgelenkstützen (8) eine Feder (5, 12) auf den Einschlagdraht (1) aufgesetzt oder dieser als Feder gebogen ist.

6.) Vorrichtung nach Anspruch 5,
**dadurch gekennzeichnet,**
daß die Feder als Kegelfeder oder als Schraubenfeder ausgebildet ist.

7.) Vorrichtung nach Anspruch 1,
**dadurch gekennzeichnet,**
daß anstelle von einem der Drehgelenke (8) eine Sperrkugel (9) vorgesehen ist.

8.) Vorrichtung nach einem oder mehreren der vorgenannten Ansprüche,
**dadurch gekennzeichnet,**
daß die Drehgelenkstützen (8) respektive die anderen Stützen (5, 9, 12) auf dem Kirschnerdraht durch Abquetschen des Drahtes, dessen winkelige Abbiegung oder mittels Aufbringen von Stecksicherungen gehalten sind.

9.) Vorrichtung nach einem oder mehreren der vorgenannten Ansprüche,
**dadurch gekennzeichnet,**
daß die Spannung des Kirschnerdrahtes über äußere Fixateure, vorzugsweise Ringfixateure, vorgenommen ist.

**Fig. 1**

**Fig. 2**

**Fig.3a**

5

**Fig.3b**

9

b

3

8

a

3

4

8

8

4

**Fig. 4**

8

12

1

**Fig. 5**

11

13

1

8

Fig. 6

Fig. 7

Fig. 8

Fig. 9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-3 211 682 (AESCULAP)<br>* Seite 8, Zeile 18 - Seite 9, Zeile 16; Figuren * | 1,8,9 | A 61 B 17/58 |
| A | | 2,3,5 | |
| | --- | | |
| Y | DE-B-1 089 116 (R. WELLER)<br>* Insgesamt * | 1,8,9 | |
| A | | 2,3,5 | |
| | --- | | |
| A | DE-A-3 200 156 (MECRON)<br>* Figur 1 * | 2,4 | |
| | --- | | |
| A,P | US-A-4 688 560 (R.J. SCHULZ)<br>* Spalte 2, Zeile 46 - Spalte 3, Zeile 20; Figuren 1-4 * | 5,6,8 | |
| | --- | | |
| A | DE-C- 908 906 (E. POHL)<br>* Seite 1, Zeile 30 - Seite 2, Zeile 31; Figuren 1-8 * | 5,6 | |
| | --- | | |
| A | US-A-3 809 075 (A.L. MATLES)<br>* Zusammenfassung * | 7,8 | RECHERCHIERTE SACHGEBIETE (Int. Cl.4) |
| | --- | | |
| A | WO-A-8 503 857 (S. SCHREIBER)<br>* Seite 5, Zeilen 8-12; Seite 5, Zeile 25 - Seite 6, Zeile 2; Figur 5 * | 7 | A 61 B |
| | --- | | |
| A | US-A-2 143 922 (E.E. LONGFELLOW)<br>* Figur 1 * | 1,9 | |
| | ----- | | |

Der vorliegende Recherchenbericht wurde für alle Patentansprüche erstellt

| Recherchenort | Abschlußdatum der Recherche | Prüfer |
|---|---|---|
| DEN HAAG | 23-09-1988 | WOLF C.H.S. |

EPO FORM 1503 03.82 (P0403)